**Europäisches Patentamt**

**European Patent Office**

**Office européen des brevets**

(19)

(11) Publication number : **0 420 757 B1**

(12) # EUROPEAN PATENT SPECIFICATION

(45) Date of publication of patent specification :
20.07.94 Bulletin 94/29

(51) Int. Cl.⁵ : **A61J 1/10**

(21) Application number : **90402674.7**

(22) Date of filing : **27.09.90**

(54) **Multiple blood bag system.**

(30) Priority : **27.09.89 JP 250940/89**

(43) Date of publication of application :
**03.04.91 Bulletin 91/14**

(45) Publication of the grant of the patent :
**20.07.94 Bulletin 94/29**

(84) Designated Contracting States :
**BE DE FR GB IT NL SE**

(56) References cited :
EP-A- 0 054 221
EP-A- 0 095 526
CA-A- 1 231 280
FR-A- 2 439 589
FR-A- 2 534 477

(73) Proprietor : **TERUMO KABUSHIKI KAISHA**
**No. 44-1, Hatagaya 2-chome,**
**Shibuya-ku**
**Tokyo 151 (JP)**

(72) Inventor : **Ishida, Noboru**
**818, Misonodaira**
**Fujinomiya-shi, Shizuoka-ken (JP)**

(74) Representative : **Gillard, Marie-Louise et al**
**Cabinet Beau de Loménie**
**158, rue de l'Université**
**F-75340 Paris Cédex 07 (FR)**

## Description

This invention relates to a multiple blood bag system having a primary bag and a sub-bag, and more particularly, to a multiple blood bag system having a blood collecting bag for collecting whole blood and a sub-bag for storing platelet concentrate (sometimes abbreviated as PC).

BACKGROUND OF THE INVENTION

For the reasons of effective utilization of blood and reduced burden to the patient, the currently used system for blood transfusion depends on the component transfusion concept that the blood collected from the donor is separated into components as by centrifugation and only the necessary component is transfused to the patient. The component transfusion can utilize blood more effectively than the prior art whole blood transfusion.

On the other hand, blood collection often uses a multiple blood bag system having a blood collecting bag and one or more sub-bags. Among the multiple bag systems, a triple bag system having a primary bag and two sub-bags is often used wherein the blood collected in the primary bag is separated into three components, erythrocyte concentrate, platelet concentrate, and platelet-poor plasma, for example, by effecting centrifugation twice.

U.S. Patent No. 4,222,379 or Japanese Patent Application Kokai No. 60464/1980 discloses a multiple blood bag system having a blood collecting bag formed of flexible polyvinyl chloride plasticized with di(2-ethylhexyl)phthalate (DEHP). This publication describes that since the plasticizer in the blood collecting bag material which can migrate into blood assists in survival of erythrocytes, but adversely affects platelets, the sub-bag(s) should be formed of a different material such as polyolefin copolymers. However, as compared with flexible polyvinyl chloride bags, bags of polyolefin and other different materials are less susceptible to centrifugation because of their hardness.

If the polyolefin or similar bag material is made thin enough to form a flexible sheet, the available softness is still poorer than the flexible polyvinyl chloride due to the material's physical properties. Thus the polyolefin bags tend to fail upon centrifugation. Also because of less flexibility than the flexible polyvinyl chloride, bags of polyolefin or similar material do not allow platelets therein to be uniformly shaken or vibrated during storage, with the platelets often losing activity after one to three days of storage.

In addition, flexible polyvinyl chloride is insufficiently compatible with polyolefins to achieve fusion or bond. There is a need for a connector as disclosed in the above-referenced publication, resulting in less efficient manufacture.

The polyolefin and similar materials are less resistant against heat than the flexible polyvinyl chloride as required upon autoclave sterilization. Sterilization at lower temperatures for a longer time also leads to a loss of manufacture. Finally, the polyolefin materials are generally more costly than the flexible polyvinyl chloride.

Japanese Patent Application Kokai No. 33661/1986 discloses a sub-bag for storing platelets formed from polyvinyl chloride plasticized with di-n-decyl phthalate (DDP). Since one exemplary sub-bag has an inner surface area of 50 $cm^2$ and contains 6 ml of platelet concentrate containing $10^6/mm^3$ of platelets, it is not intended to store as many platelets as $2 \times 10^{10}$ or more. Also, it is not indicated to combine this sub-bag with a blood collecting bag of DEHP-plasticized polyvinyl chloride. CA-A- 1 231 280 discloses a multiple blood bag system comprising a first bag which is made of a translucent, flexible, sterilizable PVC material and containing sufficient di-2-ethylhexyl (DEHP) phthalate as plasticizer so as to suppress hemolysis of blood cells on long term storage therein, and a second bag which is made of PVC containing a trimellitate as plasticizer so as to store platelet viably therein.

SUMMARY OF THE INVENTION

According to the present invention, there is provided a multiple blood bag system which is defined in claim 1.

Therefore, an object of the present invention is to provide a multiple blood bag system comprising a blood collecting bag of flexible polyvinyl chloride plasticized with di(2-ethylhexyl) phthalate and a sub-bag generally of a smaller volume wherein the sub-bag is suitable for platelet storage and readily connectible to the blood collecting bag through a tube so that the system as a whole is improved in handling and manufacture.

Preferably, the second bag comprises a part of sheet walls sealed along the periphery to define a chamber for storing platelet concentrate (PC) therein. The sheet walls have a thickness of 0.27 to 0.45 mm. The portion of the sheet walls which allows gas to permeation has an inner surface area of 100 to 450$cm^2$. The sheet walls defining the platelet storing chamber have advantageously a carbon dioxide gaz permeability of at least 3.1

ml ($CO_2$)/day.atm.ml (PC) at 30°C. Preferably, the second bag chamber has a capacity of storing at least $2x10^{10}$ platelets. The second bag is advantageously adapted to store at least one unit of platelet concentrate containing platelets in a concentration of $0.92x10^6$ to $3.4x10^6$/µl. In this specification, one unit means a platelet concentrate resulting from 200 to 450 ml of whole blood from a donor. Platelets can be stored in the second bag for an effective life of at least 72 hours.

One or more additional sub-bags can be added to the above described system. Advantageously, a third bag -formed of the same material as the first or second bad- is added. It is connected to the tube between said first and second bags through another tube.

## BRIEF DESCRIPTION OF THE DRAWINGS

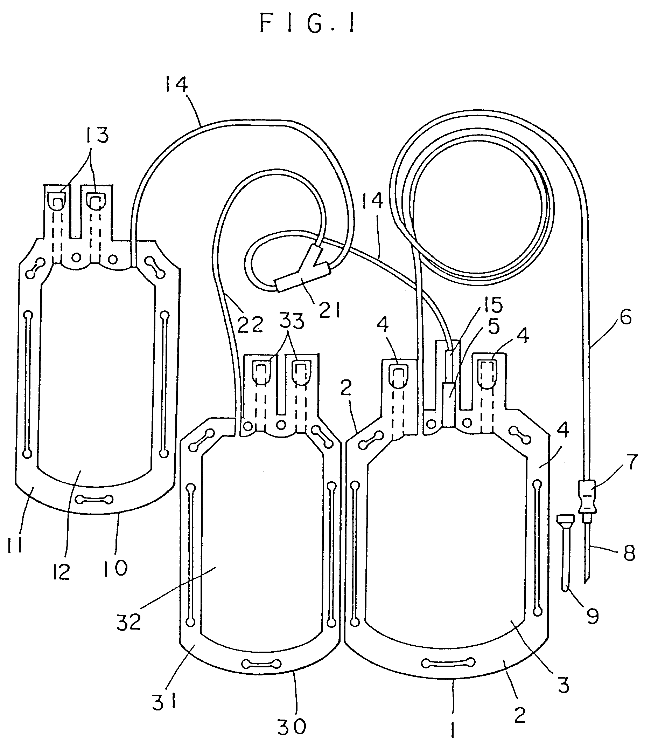

The only figure, FIG. 1 is a plan view of a multiple blood bag system according to one embodiment of the present invention.

## DETAILED DESCRIPTION OF THE INVENTION

Referring to FIG. 1, there is illustrated a triple bag system comprising a primary bag 1 for collecting blood, a sub-bag 10 for storing platelet concentrate (to be referred to as "PC storage bag", hereinafter), and an additional sub-bag 30.

The primary bag or blood collecting bag 1 shown at the right in FIG. 1 is formed from a pair of sheets of flexible polyvinyl chloride which are fusion bonded along their periphery to form a seal 2 by RF (radio frequency) heating, ultrasonic sealing or other heat sealing techniques. The sheets thus define a chamber 3 for storing a blood component within the confine of the peripheral seal 2.

It is understood that the bags, of course, can be formed from a single sheet by folding the sheet and heat sealing the mating edges and this is also true for the sub-bags.

The bag 1 at the top has two outlet ports 4 and 4 to which peelable tabs are attached and another outlet port 5 disposed therebetween for coupling to the PC storage bag 10. A length of flexible tube 6 at one end is connected to the top of the bag 1 adjacent one outlet port 4 for fluid communication with the chamber 3. The tube 6 at the other end has a blood collecting needle 8 mounted through a hub 7. A cap 9 is fitted to the hub 7 so as to enclose the needle 8 therein for projection.

The PC storage bag 10 shown at the left in the figure is also formed from a pair of sheets of flexible polyvinyl chloride which are fusion bonded along their periphery to form a seal 11 by RF heating, ultrasonic sealing or other heat sealing techniques. The sheets thus define within the confine of the peripheral seal 11 a chamber 12 for storing platelet/plasma concentrate which is separated from the blood in the primary bag 1.

The PC storage bag or sub-bag 10 at the top has two outlet ports 13 and 13 to which peelable tabs are attached. A flexible tube 14 of flexible polyvinyl chloride at one end is connected to the top of the sub-bag 10 near one outlet port 13 for fluid communication with the chamber 12. The tube 14 at the other end is connected to the outlet port 5 of the blood collecting bag 1 through a connector 15. Then the blood (component) storing chamber 3 of the blood collecting bag 1 is in flow communication with the platelet storing chamber 12 of the PC storage bag 10 through the tube 14.

In the illustrated embodiment, a branch connector 21 is provided midway the tube 14. The branch connector 21 has one branch connected to one end of a tube 22 similar to the tube 14. The other end of the tube 22 is connected to the other sub-bag 30 which, like the PC storage bag 10, defines a chamber 32 within the confine of a peripheral seal 31 and includes outlet ports 33 having peelable tabs attached thereto. When the platelet/plasma concentrate once separated from the collected whole blood by centrifugation and stored in the sub-bag 10 is again centrifuged to divide into platelet concentrate and platelet-poor plasma, the second sub bag 30 serves to collect and store the supernatant platelet-poor plasma through the tube 22.

Further, one or more additional sub-bags can be added to the system although such bags are not shown in the figure. The additional sub-bags include a bag for collecting cryoprecipitate (AHF) and a bag for collecting leukocytes.

According to the invention, the PC storage bag 10 is formed of flexible polyvinyl chloride plasticized with di-n-decyl phthalate (DDP). Flexible polyvinyl chloride is used to form the PC storage bag for the reason that it has sufficient flexibility and heat resistance to withstand centrifugal operation and sterilization. The plasticizer used in flexible polyvinyl chloride is DDP with the following benefits.

First, the DDP-plasticized polyvinyl chloride has about 1.6 times and about 2 times higher permeability of oxygen and carbon dioxide than the DEHP-plasticized polyvinyl chloride, respectively. Then a bag of the former can have a smaller volume or surface area than the latter for storing the same number of platelets. The volume reduction facilitates the operation of preparing blood components. The use of a more compact bag is advantageous with respect to centrifugal cup volume, separator stand size, the volume and size of a shaking machine used during platelet storage, transportation and storage containers, occupying space and the like. Material saving is an additional benefit on manufacture.

Second, the DDP-plasticized polyvinyl chloride minimizes the leaching of plasticizer (DDP). More particularly, the plasticizer leached into plasma is reduced to 1/70 to 1/100 as compared with the the DEHP-plasticized polyvinyl chloride. Then the migration of DEHP from the primary bag to the sub-bag is as small as about 1/12 of the DEHP level found in a bag which itself contains DEHP.

Third, di-n-decyl phthalate (DDP) itself is less toxic and mutagen-free (see Japanese Patent Application Kokai No. 33661/1986).

In general, conventional PC storage bags are formed from a pair of sheets of flexible polyvinyl chloride plasticized with di(2-ethylhexyl)phthalate (DEHP) having a thickness of 0.35 to 0.45 mm, with the sheets being peripherally sealed to define a platelet storing chamber having an inner surface area of 100 to 450 cm$^2$. Often, PC storage bags are designed to store two units of platelet concentration and thus define a platelet storing chamber having an inner surface area of 240 to 320 cm$^2$. Then the bags can accommodate at least $2 \times 10^{10}$ platelets, especially $4 \times 10^{10}$ to $9 \times 10^{10}$ platelets for an effective life of up to 72 hours. When it is desired to store more than $9 \times 10^{10}$ platelets, typically about $12 \times 10^{10}$ platelets as two units of PC, due to insufficient gas permeability of the bag-forming sheets, a pH lowering occurs to such an extent that storage of sound platelets cannot continue for 3 days.

In order that these bags accommodate an amount of PC corresponding to 400 ml of whole blood and containing a varying number of platelets between the lower and upper limits over 72 hours, the platelet storing chamber should have an increased inner surface area (e.g., about 360 to 550 cm$^2$) and/or the sheets have to be thinner, which are undesired for compactness and strength.

In contrast, the PC storage bag according to the present invention is formed from sheets of flexible polyvinyl chloride plasticized with DDP which have sufficiently increased gas permeability as previously mentioned to allow for sound storage of a desired amount of PC over 72 hours while keeping the sheet thickness and the effective inner surface area of the platelet storing chamber unchanged from the conventional design.

The sheet-forming composition preferably contains 100 parts by weight of polyvinyl chloride and 30 to 70 parts by weight of DDP plasticizer. Less than 30 parts of DDP would be too small to impart plasticity whereas more than 70 parts of DDP is less desirable because an increased amount of plasticizer will dissolve out into the bag interior and strength will be adversely affected. The composition may further contain a stabilizer and any desired other additives. Typical stabilizers are epoxy compounds and Ca-Zn stabilizers.

The sheet walls defining the platelet storing chamber 12 should preferably have a thickness of from 0.27 to 0.45 mm, more preferably from 0.30 to 0.45 mm. Sheet walls of thinner than 0.27 mm are sometimes insufficient to provide strength and invite a pH increase causing a premature loss of function of PC. Sheet walls of thicker than 0.45 mm are often undesired in that an inner surface portion of the platelet storing chamber which participates in gas permeation fails to achieve the desired gas permeation.

The portion of the platelet storing chamber 12 which allows gas permeation should have an inner surface area (effective inner surface area, essentially equal to total inner surface area minus labeling area) of from 200 to 350 cm$^2$, preferably from 260 to 340 cm$^2$. An inner surface area of less than 100 cm$^2$ is too small to provide effective gas permeation whereas more than 450 cm$^2$ naturally leads to an increased size of bag with difficulty of handling.

The sheet walls defining the platelet storing chamber 12 should preferably have a carbon dioxide gas permeability of at least 3.1 ml($CO_2$)/day·atm·ml(PC) at 30°C, more preferably at least 3.6 ml($CO_2$)/day·atm·ml (PC) at 30°C, most preferably at least 4.0 ml($CO_2$)/day·atm·ml (PC) at 30°C. A carbon dioxide gas permeability of lower than 3.1 ml($CO_2$)/day·atm·ml (PC) at 30°C is less desirable for long-term platelet storage.

With the above-controlled parameters, there is obtained a PC storage bag which can store two units of platelet concentrate, preferably an amount of platelet concentrate containing more than $9 \times 10^{10}$ platelets for an effective life of 72 hours or longer. The platelet concentrate typically contains $0.92 \times 10^6$ to $3.4 \times 10^6$ platelets per microliter ($\mu$l).

The PC storage bag according to the invention is preferably designed to store two units of platelet concentrate although the storage of more or less amounts, for example, one unit of platelet concentrate is not excluded.

The primary bag or blood collecting bag 1 is formed from flexible polyvinyl chloride plasticized with di-2-ethylhexyl phthalate (DEHP). Flexible polyvinyl chloride is used to form the primary bag for the reason that it has sufficient flexibility and heat resistance to withstand centrifugal operation and sterilization. The blood collecting bag has double functions of collecting whole blood and subsequently storing erythrocyte concentrate obtained therefrom by centrifugation. The use of DEHP plasticizer in the blood collecting bag contributes to long-term storage of erythrocytes since the DEHP plasticizer has a function to protect erythrocyte membrane.

The sheet-forming composition preferably contains 100 parts by weight of polyvinyl chloride and 30 to 70 parts by weight of DEHP plasticizer. Less than 30 parts of DEHP would be too small to impart plasticity whereas more than 70 parts of DEHP is less desirable because an increased amount of plasticizer will dissolve out into the bag interior and strength will lower. The composition may further contain a stabilizer and any desired other additives. Typical stabilizers are epoxy compounds and Ca-Zn stabilizers.

The sheet walls defining the blood component storing chamber 3 should preferably have a thickness of from 0.27 to 0.45 mm, more preferably from 0.35 to 0.45 mm. Sheet walls of thinner than 0.27 mm are sometimes insufficient to provide strength whereas with sheet walls of thicker than 0.45 mm, softness is reduced to adversely affect operations of blood collection and blood displacement upon transfusion.

Several benefits are available with the system of the present invention including a primary bag formed of DEHP-plasticized vinyl chloride and a sub-bag formed of highly gas permeable material in the form of DDP-plasticized vinyl chloride rather than polyolefin.

First, the sub-bag is sufficiently soft to avoid failure upon centrifugation as compared with the polyolefin bag.

Second, the soft sub-bag allows the platelet contents to undergo uniform vibration during storage, as compared with the polyolefin bag, contributing to sound platelet storage.

Third, the sub-bag can be easily connected to the primary or blood collecting bag of polyvinyl chloride or a tube of polyvinyl chloride, achieving a high level of fluid-tight seal as compared with the connection between a polyolefin bag and a vinyl chloride member.

Fourth, the present system enables efficient autoclave sterilization at higher temperatures because the polyvinyl chloride is generally more heat resistant than the polyolefin.

Fifth, the polyvinyl chloride is generally less costly than the polyolefin.

It should be understood that the remaining sub-bags, for example, sub-bag 30 may be formed from a resin composition which is the same as either the blood collecting bag 1 or PC storage bag 10.

The connecting tubes, for example, tubes 6, 14, 22 may also be formed from a polyvinyl chloride composition which ensures firm bonding to the bags although the exact tube composition is not particularly limited.

Next, the operation of the multiple blood bag system of the invention is described. First, whole blood (for example, about 400 ml) is collected in bag 1 through tube 6 and needle 8. The blood is then separated into erythrocyte concentrate, platelet concentrate and platelet-poor plasma in a conventional well-known manner by centrifugation.

The platelet concentrate (for example, about 40 ml) is introduced into platelet storing chamber 12 of sub-bag 10, and tube 14 is blocked by means of a clamp (not shown).

In this condition, the PC storage bag 10 is allowed to stand in the air. The effective life of platelets stored in sub-bag 10 can exceed 72 hours. The sub-bag 10 is preferably kept at room temperature, 20 to 24°C for physiological activity suppression and protection of platelets although storage at higher or lower temperatures is acceptable as the case may be.

## EXAMPLE

One example of the present invention is given below by way of illustration and not by way of limitation.

Example and Comparative Examples 1 and 2

Bags were prepared and assembled into systems while they were subjected to various tests.

1) PC storage bag

PC storage bags having the structure of bag 10 shown in the figure were prepared. These bags were formed from sheets of plasticized flexible polyvinyl chloride. The type and content of the plasticizer and the sheet thickness are reported in Table 1.

2) Blood collecting bag

Blood collecting bags having the structure of bag 1 in the figure were prepared from sheets of a polyvinyl chloride composition of 100 parts by weight of flexible polyvinyl chloride and 52 parts by weight of DEHP having a thickness of 0.39 mm. The bags each had a volume of 400 ml, an effective inner surface area of 340 cm$^2$ and an oxygen gas permeability of 40 ml($O_2$)/day·atm at 30°C.

3) Sub-bag

Second sub bags having the structure of sub-bag 30 in the figure were prepared from the same compositions as used for the blood collecting bags. The sub-bags each had a volume of 300 ml and an effective inner surface area of 280 cm².

4) Assembly of multiple blood bag system

Tubes 14 and 22 of polyvinyl chloride were bonded to the bags by RF heat sealing in the arrangement shown in the figure. The system was subjected to autoclave sterilization at 118°C for 30 minutes.

5) Gas permeability of bags

Using a fully automatic gas permeability meter, model L100-3001 (manufactured by Lyssy), the gas permeability of PC storage bag sheets were measured. Based on the measured gas permeability, sheet thickness, effective inner surface area and volume, the quantity of $CO_2$ gas permeated per ml of platelet concentrate was calculated.

6) Preparation and storage of PC

The bag 1 was charged with 56 ml of CPD liquid and 400 ml of whole blood was then collected therein. The blood was subjected to centrifugal separation at 1100G and 22°C for 6 minutes by a centrifuge model DPR-6000 (manufactured by Damon Inc.), obtaining platelet-rich plasma (PRP) which was transferred to PC storage bag 10.

The bag 1 having erythrocyte concentrate accommodated therein was removed by cutting tube 14 at a position nearer to bag 1 with respect to branch 21. By performing centrifugal separation on sub-bag 10 at 2500G and 22°C for 6 minutes, the PRP in sub-bag 10 was separated into supernatant or platelet-poor plasma (PPP) which was transferred to sub-bag 30 and platelet concentrate (PC) which was left in sub-bag 10.

Three PC storage bags of Example and Comparative Examples 1 and 2 each contained two units of PC (about 40 ml, $11 \times 10^{10}$ platelets), which was stored at 22°C for 96 hours while vibrating at 30 r.p.m. by a shaker, Eight Shaker (manufactured by Yayoi K.K.).

7) Platelet function test

A 2-ml portion was sampled out of the PC during storage after the lapse of 24, 48, 72 and 96 hours and tested for the following seven items.

(a) Platelet number (concentration)
   An automatic blood cell counter Sysmex Model CC-180 (manufactured by Toa Iyou Densi K.K.) was used.
(b) pH
   A pH meter model F8DP (manufactured by Horiba K.K.) was used.
(c) Agglomeration (ADP, collagen, and ADP + collagen) and low osmotic pressure shock recovery (%HSR)
(d) Plasma LDH activity (platelet leak)
(e) Plasma glucose concentration and plasma lactate concentration
(f) Average platelet volume (MVP)
(g) Morphology score
   The effective life of platelets was determined from overall judgment of these seven items with emphasis placed on item (b), that is, the time passed until a pH lowering occurred.
   The results are shown in Table 1.

## Table 1

| | Example | CE1 | CE2 |
|---|---|---|---|
| Plasticizer, type | DDP | DEHP | DEHP |
| content* | 52 | 52 | 52 |
| Sheet thickness, mm | 0.39 | 0.39 | 0.30 |
| Effective inner surface area ($cm^2$) | 280 | 280 | 380 |
| Bag volume (ml) | 300 | 300 | 500 |
| PC (units) | 2 | 2 | 2 |
| $CO_2$ gas permeability $ml(CO_2)/day \cdot atm \cdot ml(PC)$ | 9.2 | 4.6 | 9.2 |
| Effective life (hour) | 72 | 24 | 72 |

\* parts by weight per 100 parts by weight of flexible polyvinyl chloride

As seen from Table 1, the PC storage bag of Example using DDP plasticizer allowed two units of PC containing as many as $11 \times 10^{10}$ platelets to be stored for 72 hours due to improved gas permeability. The bag of Comparative Example 1 using DEHP plasticizer allowed an amount of PC containing the same number of platelets to be stored for only 24 hours due to poor gas permeability. The bag of Comparative Example 2 using DEHP plasticizer, but an increased effective inner surface area of 380 $cm^2$ allowed an amount of PC containing the same number of platelets to be stored for 72 hours. However, this bag was of a larger size with some inconvenience in handling.

There has been described a multiple blood bag system including a primary bag for collecting blood and a sub-bag for storing platelet concentrate, the sub-bag being of a relatively small size, but capable of storing at least $2 \times 10^{10}$ platelets for an extended time of 72 hours or longer. The system is quite useful in practice owing to several advantages in bonding, handling, and manufacture.

It is thought that the present invention and many of its attendant advantages will be understood from the foregoing description and it will be apparent that various changes may be made in the form, construction and arrangement of the parts thereof without departing from the scope of the invention as specified in the following claims, or sacrificing all of its material advantage, the forms hereinbefore described being merely preferred or exemplary embodiments thereof.

## Claims

1. A multiple blood bag system comprising :
   a first bag (1) made of flexible polyvinylchloride plasticized essentially with di(2-ethylhexyl)phtalate ;
   a second bag (10) made of flexible polyvinylchloride and a flexible tube (14) made of polyvinylchloride connecting the first and second bags in a fluid flow relationship, characterised in that said second bag is plasticized essentially with di-n-decylphtalate and comprises a pair of sheet walls sealed along the periphery to define a chamber for storing more than $2 \times 10^{10}$ platelets therein, the portion of the sheet walls which allows gas permeation having an inner surface area of 200 to 350 $cm^2$.

2. The system of claim 1 wherein the sheet walls have a thickness of 0.27 to 0.45 mm, and the portion of the sheet walls which allows gas permeation have an inner surface area of 260 to 340 $cm^2$.

7

3. The system of claim 2 wherein the sheet walls defining the platelet storing chamber have a carbon dioxide gas permeability of at least 3.1 ml ($CO_2$)/day·atm·ml (PC) at 30°C.

4. The system of any one of claims 1 to 3 wherein said second bag is adapted to store at least one unit of platelet concentrate containing platelets in a concentration of $0.92 \times 10^6$ to $3.4 \times 10^6$/μl.

5. The system of any one of claims 1 to 4 wherein platelets are stored in said second bag for an effective life of at least 72 hours.

6. The system of any one of claims 1 to 5 which further comprises a third bag (30) which is formed from the same material as the first or second bag and connected to the tube (14) through another tube (22).


**Patentansprüche**

1. Mehrteiliges Blutbeutelsystem, umfassend:
einen ersten Beutel (1) aus flexiblem, mit im wesentlichen Di(2-Ethylhexyl)phthalat plastifiziertem Polyvinylchlorid;
einen zweiten Beutel (10) aus flexiblem Polyvinylchlorid und einen flexiblen Schlauch (14) aus Polyvinylchlorid, der den ersten und zweiten Beutel in einer Flüssigkeitsstrombeziehung verbindet, dadurch gekennzeichnet, daß der zweite Beutel mit im wesentlichen Di-n-Decylphthalat plastifiziert ist und ein Paar Folienwände umfaßt, die entlang der Außenfläche versiegelt sind, wobei eine Kammer zum Lagern von mehr als $2 \times 10^{10}$ Thrombozyten in der Kammer eingegrenzt wird und der Teil der Folienwände, der Gasdurchlässigkeit aufweist, eine Innenfläche von 200 bis 350 cm² hat.

2. System nach Anspruch 1, bei dem die Folienwände eine Dicke von 0,27 bis 0,45 mm haben und der Teil der Folienwände, der Gasdurchlässigkeit aufweist, eine Innenfläche von 260 bis 340 cm² hat.

3. System nach Anspruch 2, bei dem die die Thrombozyten-Lagerkammer eingrenzenden Folienwände eine Kohlendioxidgas-Durchlässigkeit von mindestens 3,1 ml ($CO_2$)/Tag·atm·ml (PC) bei 30 °C aufweisen.

4. System nach einem der Ansprüche 1 bis 3, bei dem der zweite Beutel an die Lagerung von mindestens einer Einheit Thrombozyten-Konzentrat mit Thrombozyten in einer Konzentration von $0,92 \times 10^6$ bis $3,4 \times 10^6$ /μl angepaßt ist.

5. System nach einem der Ansprüche 1 bis 4, bei dem Thrombozyten in dem zweiten Beutel für eine effektive Lebensdauer von mindestens 72 Stunden gelagert werden.

6. System nach einem der Ansprüche 1 bis 5, das ferner einen dritten Beutel (30) umfaßt, der aus demselben Material wie der erste oder zweite Beutel gebildet und mit dem Schlauch (14) durch einen weiteren Schlauch (22) verbunden ist.


**Revendications**

1. Système de sacs à sang multiples comprenant :
un premier sac (1) constitué par du poly(chlorure de vinyle) flexible plastifié essentiellement avec du phtalate de di(2-éthylhexyle) ;
un second sac (10) constitué par du poly(chlorure de vinyle) flexible ;
et
un tube flexible (14) constitué par du poly(chlorure de vinyle) qui relie les premier et second sacs par écoulement de fluide, caractérisé en ce que ledit second sac est plastifié essentiellement avec du phtalate de di-n-décyle et comprend une paire de parois en feuille soudées suivant la périphérie pour définir une chambre destinée à stocker plus de $2 \times 10^{10}$ plaquettes, la partie des parois en feuille qui permet aux gaz de la traverser ayant une surface interne d'une aire de 200 à 350 cm².

2. Système selon la revendication 1, dans lequel les parois en feuille ont une épaisseur de 0,27 à 0,45 mm, et la partie des parois en feuille qui permet aux gaz de la traverser a une surface interne d'une aire de 260 à 340 cm².

3. Système selon la revendication 2, dans lequel les parois en feuille qui définissent la chambre de stockage des plaquettes ont une perméabilité au dioxyde de carbone d'au moins 3,1 ml $(CO_2)$/j.atm.ml (CP) à 30°C.

4. Système selon l'une quelconque des revendications 1 à 3, dans lequel ledit second sac est conçu pour stocker au moins une unité de concentré de plaquettes contenant des plaquettes en une concentration de $0,92 \times 10^6$ à $3,4 \times 10^6$/$\mu$l.

5. Système selon l'une quelconque des revendications 1 à 4, dans lequel les plaquettes sont stockées dans ledit second sac pendant une durée de vie efficace d'au moins 72 h.

6. Système selon l'une quelconque des revendications 1 à 5, qui comprend en outre un troisième sac (30) qui est constitué par la même matière que le premier ou second sac et qui est relié au tube (14) par un autre tube (22).

FIG.1